# EUROPEAN PATENT APPLICATION

(11) **EP 2 196 204 A1**
(43) Date of publication of application: **16.06.2010**
(21) Application number: 08715150.2
(22) Date of filing: 05.03.2008
(51) Int. Cl.: A61K 31/365, C07D 493/22, A61K 36/06, A61P 35/00

(54) **MYROTHECIUM SP. MYCELIAL EXTRACT FOR INHIBITING TUMOR CELLS GROWTH**

(30) Priority: 08.10.2007 CN 200710162725
(71) Applicant: Golden Biotechnology Corporation, Taiwan (CN)
(72) Inventor: LIU, Sheng-Yun, Taiwan (CN); WEN, Wu-Che, Taiwan (CN); KUO, Mao-Tien, Taiwan (CN)
(74) Representative: Winkler, Andreas Fritz Ernst
(86) International application number: PCT/CN2008/070414
(87) International publication number: WO 2009/046640

(57) **Abstract**

The present invention relates to a tumor-inhibiting compound, in particular to verrucarin A and verrucarin J isolated from *Myrothecium* sp., and its use in inhibiting the growth of cancer cell including lung cancer, hepatic cancer and prostate cancer. Verrucarin A and verrucarin J in the present invention were applied in inhibiting the growth of human hepatic cancer cell lines Hep3B and HepG2, human lung cancer cell line A549, and human prostate cancer cell lines LNCaP and DU-145, which can further be used as pharmaceutical compositions for inhibition of cancers including hepatic cancer, lung cancer and prostate cancer.

## Description

### BACKGROUND OF THE INVENTION

### 1. Field of the Invention

The present invention relates to a method for using a compound to inhibit the growth of cancer cells, in particular to compounds isolated and purified from the mycelium of *Myrothecium* sp. which can be applied in inhibiting the growth of lung cancer cells, hepatic cancer cells and prostate cancer cells.

### 2. The Prior Arts

Cancer has become the number one killer disease after the 20th century. According to statistics made by the department of Health in Taiwan in June 2002, malignant cancer has been the first leading cause on top 10 causes of death for the last 20 years since 1982, with a mortality rate of 147.68 per 100,000 populations, and approximately 33,000 deaths in 2001. Searches for effective anti-cancer compounds with mild side effects therefore become imperative.

Trichothecenes belong to the family of sesquiterpenoids with broad physiological activities, which are produced by several fungi including *Fusarium, Trichothecium, Trichoderma, Myrothecium, Cephalosporium, Stachybotrys,* and *Cylindrocarpon.* Studies have shown that trichothecenes exert anti-bacterial activity, and have cytostatic activity *in vitro.* Trichothecenes can be divided into two groups including macrocyclic and non-macrocyclic compounds based on the differences in chemical structure. Mycotoxins containing macrocyclic trichothecenes such as verrucarins, roridins, satratoxin, vertisporin, and baccharinoid are famous and reported to have a variety of biological activities. They inhibit initiation of protein translation further interfere with protein synthesis by binding with polysomes and 80S ribosomes in eukaryotic cells. Most of these mycotoxins have been applied in antibiotics to inhibit bacterial growth, in antiflammation and in immunomodulatory. Verrucarins, also known as muconomycin and produced by *Myrothecium* sp., can be categorized into verrucarin A (muconomycin A), verrucarin J (muconomycin J), verrucarin K and the like according to their chemical structures. Verrucarins have been shown to exert prolonged inhibition of protein and glycoprotein synthesis and have immuno-suppressive activity. Antiviral activities against Newcastle virus and tobacco mosaic virus of verrucarins were also reported.

Inhibition of lung cancer, hepatic cancer and prostate cancer were not studied in the abovementioned applications of verrucarins. Therefore, the application of verrucarins in cancers can be great benefits to the therapies of lung cancer, hepatic cancer and prostate cancer in human.

### SUMMARY OF THE INVENTION

In order to identify the anti-tumor compounds, the invention relates to compounds with the following structural formula isolating and purifing from the extracts of *Myrothecium* sp..

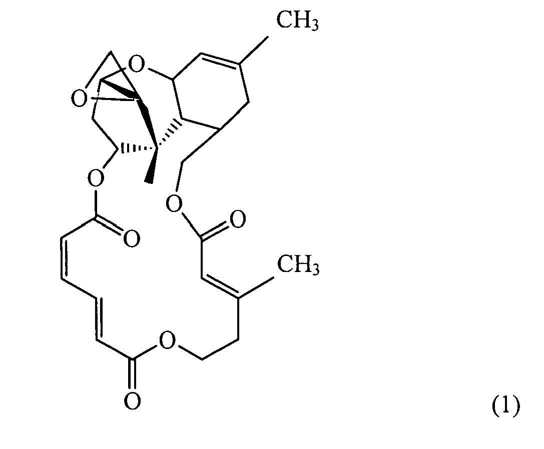

The compound of formula (1) is verrucarin A (muconomycin B), with a molecular formula of C₂₇H₃₂O₈, and a molecular weight of 484.

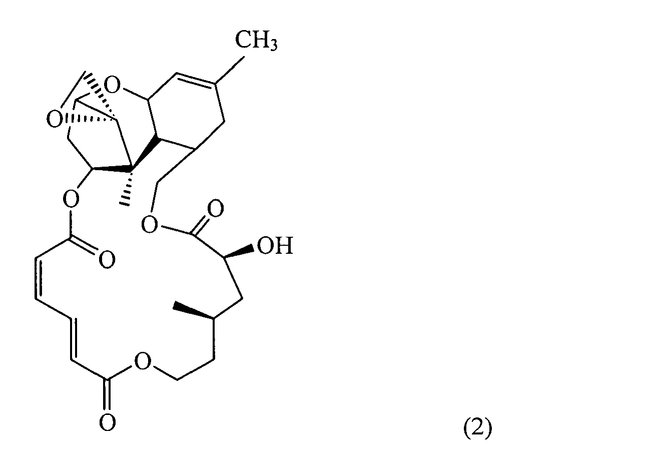

The compound of formula (2) is verrucarin J (muconomycin B), with a molecular formula of C₂₇H₃₄O₉, and a molecular weight of 502.

The compounds of formula (1) and formula (2) in the present invention are purified from organic solvent extracts of *Myrothecium* sp. The organic solvents used in the present invention include, but are not limited to, alcohols such as methanol, ethanol or propanol, with ethanol being preferred. Any other suitable organic liquids which can extract compounds of formula (1) or formula (2) including esters such as ethyl acetate, alkanes such as hexane, or haloalkanes such as chloromethane, chloroethane can be applied.

The present invention provides a method for using abovementioned compounds of formula (1) and formula (2) to inhibit tumor cell growth. More specifically, the method comprises administrating an effective dose of the compounds to inhibit a range of cancer cells including lung cancer, hepatic cancer and prostate cancer, which result in slowering the growth of cancer cells, further inhibiting proliferation of cancer cells and decreasing the risk of malignancy. Therefore the method for using the compound of the present invention can be applied in the treatment of lung cancer, hepatic cancer, prostate cancer and more.

The present invention further provides a method of using a pharmaceutical composition containing an effective dose of the compounds of structural formula (1) and structural formula (2) for treating lung cancer, hepatic cancer and prostate cancer to enhance the therapeutic effects against cancer cells.

The compounds of formula (1) and/or formula (2) in the invention can be incorporated into the pharmaceutical compositions for treating lung cancer, hepatic cancer, and prostate cancer to inhibit the growth of tumor cells. The pharmaceutical compositions include not only the compounds of formula (1) and/or formula (2), but also the pharmaceutically accepted carriers. The carriers include, but are not limited to, excipients such as water, fillers such as sucrose or starch, binders such as cellulose derivatives, diluents, disintegrants, absorption enhancers or sweeteners. The inventive composition can be manufactured through mixing the compounds of formula (1) and/or formula (2) with at least one of the carriers by means of conventional methods known in the pharmaceutically technical field, which can be formulated, but are not limited to, as a powder, tablet, capsule, pellets, granules or other liquid formulation.

The present invention is further explained in the following embodiment illustration and examples. Those examples below should not, however, be considered to limit the scope of the invention, it is contemplated that modifications will readily occur to those skilled in the art, which modifications will be within the spirit of the invention and the scope of the appended claims.

### DETAILED DESCRIPTION OF THE PREFERRED EMBODIMENT

The mycelia of *Myrothecium* sp. were cultivated and harvested. The mycelia were then extracted with organic solvents to obtain the organic solvent extracts of *Myrothecium* sp by the well known extraction methods in the art. The organic solvents include, but not limited to, alcohols such as methanol, ethanol or propanol, esters such as ethyl acetate, alkanes such as hexane, or haloalkanes such as chloromethane, chloroethane. Among them, alcohol is preferred, and 95% ethanol is particularly preferred.

The organic solvent extracts of *Myrothecium* sp. were subjected to preparative high-performance liquid chromatography (HPLC) for isolation and purification. Each fraction was recovered and assayed for biological activities. The potent fractions with anti-cancer effects were analyzed for the composition and further assayed against different tumor cells. The above approach then led to the identification of compounds of the formula (1) and formula (2), which inhibited the growth of tumor cells of lung cancer, hepatic cancer, and prostate cancer.

The compounds of formula (1) and formula (2) were demonstrated with anti-cancer effects using 3-(4,5-dimethylthiazol-2-yl)-2,5-diphenyltetrazolium bromide (MTT) assay according to the anti-tumor agents screening model of National Cancer Institute (NCI) of the United States National Institutes of Health on cell survival rates using cell lines of lung cancer, hepatic cancer, prostate cancer and the like. The above assays had proved that verrucarin A and verrucarin J decreased survival rates of lung cancer cell lines (A-549), hepatic carcinoma cell lines (Hep 3B and Hep G2) and prostate cancer cell lines (LNCaP and DU-145), at the same time showed relatively low maximal inhibitory concentration (IC₅₀) values. Therefore verrucarin A and verrucarin J can be used in inhibiting cancer cell growth of lung cancer, hepatic cancer, and prostate cancer, which can further be applied in cancer treatment of lung cancer, hepatic cancer, prostate cancer and the like. The details of the examples are described as follows:

### Example 1

### Cultivation of Myrothecium sp.

Media for *Myrothecium* sp. were prepared as described below. The media contained 0.1-2 g of NaCl, 5-10g of peptone, 1-2 g of yeast extract, 3-10 g of agar, 3-10 g of cereal (chosen from rice, wheat, glutinous rice or corn and the like), 1-2 g of nitrogen source (NH₄NO₃ or KNO₃), 1-3 g of carbon source (glucose, fructose or sucrose), some phosphate salt, trace amount of metal ions (magnesium, or calcium) in 800-1000 ml of double distilled water. Final pH was adjusted to 6.5-7.5 with 1 N NaOH or 1 N HCl. Media were autoclaved at 120°C for 20 min in a conical flask with cotton plugging. *Myrothecium verrucaria* or *Myrothecium rodium tode* was inoculated into the cooled media in a laminar flow hood and incubated at 20-30°C for 4-8 weeks to gain the mycelia of *Myrothecium* sp.. *Myrothecium* sp. were collected from soil under trees in hill side of Snow Mountain, Taiwan and identified by Professor Tseng, Hsien-hsiung of National Taiwan University. Microbial sources of interest also include, but are not limited to the abovementioned strains, any alternative strains used to provide verrucarin A and veccucarin J.

### Example 2

### Isolation of compounds with anti-cancer activities

One hundred grams of mycelia of *Myrothecium sp.* from Example 1 were placed into a flask. A proper amount of 95% alcohol was added into the flask and stirred at 20-25°C for at least 1 hour. The solution was filtered through a filter with 0.45 µm membrane and the filtrate was collected as the extract. The organic solvents used include, but are not limited to, alcohols such as methanol, ethanol or propanol, with ethanol being preferred.

The filtrate of *Myrothecium* sp. was subjected to High Performance Liquid chromatography (HPLC) analysis with a silica gel column. The mobile phase consisted of hexane and ethyl acetate (EA). The column effluent was monitored with a UV-visible detector. Ten fractions were collected and assayed for their biological activities. Compositions of fractions containing biological activities were analyzed. Separation with different ratios of hexane and acetone on these fractions yielded compounds of formula (1) and formula (2). Compound of formula (1) is verrucarin A after analysis, which showed a molecular formula of C₂₇H₃₂O₈, a molecular weight of 484. Compound of formula (2) is verrucarin J after analysis, which showed a molecular formula of C₂₇H₃₄O₉, a molecular weight of 502.

### Example 3

### In vitro assay of anti-tumor activity of hepatic cancer

The NCI anti-cancer agents screen model was employed to test the anti-cancer effect of the verrucarin A and verrucarin J in the invention. The verrucarin A and verrucarin J isolated from example 2 were added into the culture media of human hepatic cancer cells, Hep 3B or Hep G2, for determining tumor cell survival by MTT assay. MTT assay was known to assess the survival rates of cell. Hepatic cancer cells Hep 3B (under an accession number of BCRC 60434) and Hep G2 (under an accession number of BCRC 60025), were obtained from the Bioresources Collection and Research Center (BCRC) of the Food Industry Research and Development Institute.

MTT assay is commonly used to analyze cell proliferation, percentage of viable cells, and cytotoxicity. MTT (3-[4,5-dimethylthiazol-2-yl]-2,5-diphenyl tetrazolium bromide) is a yellow dye, which can be absorbed by the living cells and be reduced to violet insoluble formazan products by succinate tetrazolium reductase in mitochondria. Formation of formazan products in viable cells can therefore be used to assess and determine the survival rate of cells.

The human hepatic cancer cells, Hep 3B and Hep G2, were cultured in media containing fetal bovine serum for 24 hours. The proliferated cells were washed once with PBS, then treated with 1x trypsin-EDTA and centrifuged at 1,200 rpm for 5 minutes. The supernatant was discarded and the cell pellet was resuspended in 10 ml of fresh culture medium by gently shaking. The cells were placed in a 96-well plate. Both crude extracts of *Myrothecium* sp. (the control group, total ethanol extracts of *Myrothecium* sp. without HPLC purification), or verrucarin A and verrucarin J (the experimental group) were added into each of the wells at the following concentrations: 100, 30, 10, 3, 1, 0.3, 0.1 and 0.03 ng/ml, respectively. The cells were incubated at 37 °C in a 5% CO₂ incubator for 48 hours. MTT dye was added in a concentration of 2.5 mg/ml into each well in dark and incubated for 4 hours, followed by the addition of 100 µl of lysis buffer to stop the reaction. Subsequently, absorption was measured on an enzyme immunoassay analyzer at 570 nm for the measurement of viable cell number and determine the survival rates. The half maximal inhibitory concentration (IC₅₀) values of the control group and the experimental group were also calculated and listed in Table 1.

**Table 1 The result of in vitro survival assay for anti-tumor activity of hepatic cancer cells**

| **Samples** | | IC₅₀(ng/ml) | |
|---|---|---|---|
| | | Hep3B | HepG2 |
| Control group | crude extracts of *Myrothecium sp.* | 23.81 | 45.89 |
| Experimental | verrucarin A | 2.31 | 10.21 |
| group | verrucarin J | 3.12 | 13.31 |

From the result of table 1, verrucarin A and verrucarin J can effectively decrease the survival rates of human hepatic cancer cell line Hep 3B and Hep G2. The IC₅₀ values of verrucarin A and verrucarin J toward Hep 3B were 2.31 ng/ml and 3.12 ng/ml respectively, which were 90.3% and 86.9% lower than the IC₅₀ values 23.81 ng/ml of crude extracts from *Myrothecium* sp.. The IC₅₀ values of verrucarin A and verrucarin J toward Hep G2 were 10.21 ng/ml and 13.31 ng/ml respectively, which were 77.75% and 71% lower than that of crude extracts from *Myrothecium* sp. In addition, hepatic cancer cells treated with verrucarin A showed lower IC₅₀ values than cells treated with verrucarin J, that is, lower concentration of verrucarin A was needed in inhibiting growth of half of the cancer cells. Verrucarin A has better inhibiting effect than verrucarin J on the growth of hepatic cancer cells.

### Example 4

### In vitro survival assay for anti-tumor activity of lung cancer

The NCI anti-cancer angents screen model was also employed to test the anti-lung cancer effect of the verrucarin A and verrucarin J in the invention. Verrucarin A and verrucarin J isolated from example 2 were added into the culture media of human lung cancer cell A549 for tumor cell survival assay, and lung cancer cell survival rates were analyzed with the abovementioned MTT assay. Lung cancer cell A549 was obtained from the Bioresources Collection and Research Center (BCRC) of the Food Industry Research and Development Institute with an accession number of BCRC 60074.

The human lung cancer cell A549 was cultured in media containing fetal bovine serum for 24 hours. The proliferated cells were washed once with PBS, then treated with 1x trypsin-EDTA and centrifuged at 1,200 rpm for 5 minutes. The supernatant was discarded and the cell pellet was resuspended in 10 ml of fresh culture medium by gently shaking. The cells were placed in a 96-well plate. Both crude extracts of *Myrothecium* sp. (the control group, total ethanol extracts of *Myrothecium* sp. without HPLC purification), or verrucarin A and verrucarin J (the experimental group) were added into each of the wells at the following concentrations: 100, 30, 10, 3, 1, 0.3, and 0.1 ng/ml, respectively. The cells were incubated at 37°C in a 5% CO₂ incubator for 48 hours. MTT dye was added in a concentration of 2.5 mg/ml into each well in dark and incubated for 4 hours, followed by the addition of 100 µl of lysis buffer to stop the reaction. The plates were read on an ELISA reader at wavelength of 570 nm to determine the survival rates. The half maximal inhibitory concentration (IC₅₀) values were also calculated and listed in Table 2.

**Table 2 The result of in vitro survival assay for anti-tumor activity of lung cancer cells**

| **Samples** | | IC₅₀(ng/ml) |
|---|---|---|
| | | A549 |
| Control group | crude extracts of *Myrothecium* sp. | 52 |
| Experimental | verrucarin A | 1.12 |
| group | verrucarin J | 2.34 |

From the result of table 2, verrucarin A and verrucarin J can effectively decrease the survival rates of human lung cancer cell line A549. The IC₅₀ values of verrucarin A and verrucarin J toward Hep 3B were 1.12 ng/ml and 2.34 ng/ml respectively, which were 97.85% and 95.5% lower than that of crude extracts from *Myrothecium* sp. (52 ng/ml). Both verrucarin A and verrucarin J showed remarkably reduced IC₅₀ values than the control group. Therefore, verrucarin A and verrucarin J can be applied in inhibiting the growth of lung cancer cells. In addition, lung cancer cells treated with verrucarin A showed lower IC₅₀ values than cells treated with verrucarin J, that is, lower concentration of verrucarin A was needed in inhibiting the growth of half of lung cancer cells. This represents that verrucarin A has better inhibiting effect than verrucarin J on the growth of lung cancer cells.

### Example 5

### In vitro survival assay for anti-tumor activity of prostate cancer

According to anti-tumor agents screening model of NCI of the United States National Institutes of Health, the abovementioned MTT assay is processed by adding Verrucarin A and verrucarin J into the culture medium of human prostate cancer tumor cells LNCaP and DU-145, respectively.

Prostate cancer is a cancer originated from epithelial cells of prostate gland, which depends on androgen in the early stage. Initially all prostate cancer cells are androgen-dependent, which can be treated with androgen-deprivation therapy. LNCaP represents this type of prostate cancer at an early stage. However, 30% of the patients will experience recurrence, and the secreting amounts of androgen become very low. Finally, recurrent cancer progresses to the androgen-independent prostate cancer, which has no effective treatment so far. DU-145 represents this type of cancer cells. Both LNCaP and DU-145 were ordered from the Bioresources Collection and Research Center (BCRC) of the Food Industry Research and Development Institute with accession numbers of CCRC 60088 and CCRC 60348 respectively.

The human hepatic cancer cells, LNCaP and DU-145, were cultured in media supplemented with fetal bovine serum for 24 hours. The proliferated cells were washed once with PBS, then treated with 1x trypsin-EDTA and centrifuged at 1,200 rpm for 5 minutes. The supernatant was discarded and the cell pellet was resuspended in 10 ml of fresh culture medium by gently shaking. The cells were placed in a 96-well plate. Water (negative control group), 4.2 ng/ml of taxol (positive control), 100, 10, 1, 0.1, and 0.01 ng/ml of crude extracts of *Myrothecium* sp. (experimental control group, crude extracts of *Myrothecium* sp. without HPLC purification) or 100, 10, 1, 0.1, 0.01 and 0.001 ng/ml of verrucarin A and verrucarin J (experimental group) were added into each of the 96 wells respectively. The cells were incubated at 37°C in a 5% CO₂ incubator for 48 hours. MTT dye was added in a concentration of 2.5 mg/ml into each well in dark and incubated for 4 hours, followed by the addition of 100 µl of lysis buffer to stop the reaction. The plates were read on an ELISA reader at wavelength of 570 nm to determine the survival rates (%). The half maximal inhibitory concentration (IC₅₀) values were also calculated and listed in Table 3 and Table 4.

**Table 3 The result of in vitro survival assay for anti-tumor activity of prostate cancer cells**

| **Samples** | | IC₅₀(ng/ml) | |
|---|---|---|---|
| | | LNCaP | DU-145 |
| Experimental control group | crude extracts of *Myrothecium* sp. | 29.31 | 33.16 |
| Experimental group | verrucarin A | 2.85 | 0.02 |
| | verrucarin J | 0.31 | 0.28 |

From the result of table 3, the cell survival rates of LNCaP and DU-145 were effectively reduced through the functions of verrucarin A and verrucarin J. The IC₅₀ values of verrucarin A and verrucarin J toward LNCaP were 2.85 ng/ml and 0.31 ng/ml respectively, which were 90.28% and 98.94% relatively lower than the IC₅₀ value of experimental control group (29.31 ng/ml). The IC₅₀ values of verrucarin A and verrucarin J toward DU-145 were 0.02 ng/ml and 0.28 ng/ml respectively, which were 99.93% and 99.16% relatively lower than the IC₅₀ value of experimental control group (33.16 ng/ml). Therefore verrucarin A and verrucarin J from *Myrothecium sp.* can be applied to inhibit the growth of prostate cancer cells, which showed much lower IC₅₀ values than the crude extracts (29.31 ng/ml and 33.16 ng/ml). In addition, LNCaP prostate cancer cells treated with verrucarin J showed lower IC50 values than verrucarin A treated cells, that is, verrucarin J has better inhibiting effect than verrucarin A on the growth of LNCaP prostate cancer cells. While DU-145 prostate cancer cells treated with verrucarin A showed lower IC50 values than verrucarin J treated cells, that is, verrucarin A has better inhibiting effect than verrucarin J on the growth of DU-145 prostate cancer cells.

**Table 4 Effects of verrucarin A and verrucarin J on the growth of DU-145 prostate cancer cell**

| samples | | concentration (ng/ml) | Cell survival rate (%) |
|---|---|---|---|
| Negative control group | water | | 100 |
| Positive control | Taxol | 4.2 | 63.88 |
| | | 100 | 20.60 |
| Experimental | crude extracts of | 10 | 91.01 |
| control group | *Myrothecium* sp. | 1 | 94.30 |
| | | 0.1 | 101.31 |
| Experimental group | | 100 | 16.96 |
| | | 10 | 17.07 |
| | verrucarin A | 1 | 16.99 |
| | | 0.1 | 21.01 |
| | | 0.01 | 72.31 |
| | | | |
| | | 0.001 | 94.33 |
| | | 100 | 20.36 |
| | | 10 | 18.95 |
| | verrucarin J | 1 | 19.93 |
| | | 0.1 | 81.11 |
| | | 0.01 | 101.30 |
| | | 0.001 | 100.21 |

Alternatively, Table 4 shows the growth of DU-145 prostate cancer cell was effectively reduced through the function of verrucarin A and verrucarin J. In comparison to the control groups, the cancer cell survival rates were reduced to 20% when verrucarin A and verrucarin J were employed in the concentration of 1 ng/ml to 100 ng/ml. The cell survival rates reduced in accordance with the addition of concentration of crude extracts of *Myrothecium* sp., or verrucarin A and verrucarin J. On the contrary, the cell survival rates increased when the employment concentration reduced. When the concentration of control and experimental group reduced to 10 ng/ml, the cell survival rate increased to 91.01% with the addition of crude extracts of *Myrothecium* sp., while the cell survival rates stayed around 19% with the addition of purified verrucarin A or verrucarin J respectively. Verrucarin A and verrucarin J were shown to be the active ingredients for inhibition of DU-145 prostate cancer cells. In addition, the cell survival rate was 63.88% when treating with 4.2 ng/ml Taxol, while the cell survival rates reduced remarkably lower to 20% when employment concentration of verrucarin A and verrucarin J were ranged from 1 ng/ml to 10 ng/ml. This demonstrated a superior inhibition effect in human prostate cancer cell of verrucarin A and verrucarin J compared to Taxol. And significant cancer inhibiting effects were observed even when the employment concentration reached to as low as 0.1 ng/ml and 1 ng/ml.

On the other hand, the cell survival rates were similar when employment concentration of verrucarin A and verrucarin J were above 1 ng/ml (ranged from 1 ng/ml to 10 ng/ml). That is, both of them showed similar anti-cancer effects within these concentration ranges. However, verrucarin A has better effect than verrucarin J in inhibition on the growth of DU-145 prostate cancer cells when the employment concentration was lowered than 0.1 ng/ml (ranged from 0.001 ng/ml to 0.1 ng/ml).

In summary, verrucarin A and verrucarin J purified from extracts of *Myrothecium* sp. can effectively inhibit the growth of prostate cancer cells LNCaP and DU-145 with different characteristics. Therefore, both verrucarin A and verrucarin J can not only be applied in inhibiting the growth of androgen-dependent prostate cancer cell LNCaP, but also can be applied in inhibiting the growth of androgen-independent prostate cancer cell DU-145. This will be beneficial to the therapy of prostate cancer and recurrent prostate cancer.

On the other hand, verrucarin A and verrucarin J can be incorporated into pharmaceutical compositions. The pharmaceutical compositions include not only the active compound verrucarin A and verrucarin J, but also the pharmaceutically accepted carriers. Examples of such carriers include, but are not limited to, excipients such as water, fillers such as sucrose or starch, binders such as cellulose derivatives, diluents, disintegrants, absorption enhancers or sweeteners. The inventive composition can be manufactured through mixing the compound of verrucarin A and verrucarin J from *Myrothecium* sp. with at least one of the carriers by means of conventional methods known in the pharmaceutically technical field, which can be formulated in the forms of powder, tablets, capsules, pellets, granules or other liquid formulation, but are not limited to. The purpose in the present invention for treatment of tumor and inhibiting the growth of cancer cells can then be accomplished.

## Claims

1. A method for inhibiting cancer cells, comprising:
administering an effective dose of a compound having the following formula to inhibit the growth of hepatic cancer cells, lung cancer cells or prostate cancer cells.

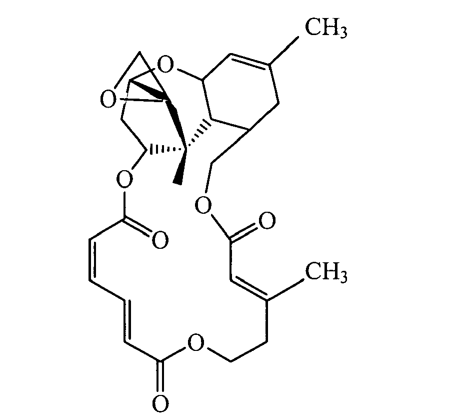

2. The method as claimed in claim 1, wherein the compound is isolated from the mycelium of *Myrothecium* sp.

3. The method as claimed in claim 1, wherein the hepatic cancer cells are from a Hep3B cell line or a HepG2 cell line.

4. The method as claimed in claim 3, wherein the half maximal inhibitory concentration (IC₅₀) for the hepatic cancer cell line Hep3B is 2.31 ng/ml.

5. The method as claimed in claim 3, wherein the half maximal inhibitory concentration (IC₅₀) for the hepatic cancer cell line HepG2 is 10.21 ng/ml.

6. The method as claimed in claim 1, wherein the lung cancer cells are from a A549 cell line.

7. The method as claimed in claim 6, wherein the half maximal inhibitory concentration (IC₅₀) for the lung cancer cell line A549 is 1.12 ng/ml.

8. The method as claimed in claim 1, wherein the prostate cancer cells are from a LNCaP cell line or a DU-145 cell line.

9. The method as claimed in claim 8, wherein the half maximal inhibitory concentration (IC₅₀) for the prostate cancer cell line LNCaP is 2.85 ng/ml.

10. The method as claimed in claim 8, wherein the half maximal inhibitory concentration (IC₅₀) for the prostate cancer cell line DU-145 is 0.02 ng/ml.

11. A pharmaceutical composition used for inhibiting the growth of cancer cells, which comprises an active dose of the compound as claimed in claim 1 and a pharmaceutically-acceptable carrier, wherein the cancer cells are selected from the group consisting of hepatic cancer, lung cancer or prostate cancer.

12. A method for inhibiting cancer cells, comprising:
administering an effective dose of a compound having the following formula to inhibit the growth of hepatic cancer cells, lung cancer cells or prostate cancer cells.

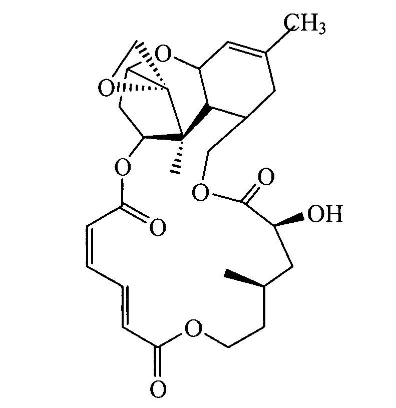

13. The method as claimed in claim 12, wherein the compound is isolated from the mycelium of *Myrothecium* sp.

14. The method as claimed in claim 12, wherein the hepatic cancer cells are from a Hep3B cell line or a HepG2 cell line.

15. The method as claimed in claim 14, wherein the half maximal inhibitory concentration (IC₅₀) for the hepatic cancer cell line Hep3B is 3.12 ng/ml.

16. The method as claimed in claim 14, wherein the half maximal inhibitory concentration (IC₅₀) for the hepatic cancer cell line HepG2 is 13.31 ng/ml.

17. The method as claimed in claim 12, wherein the lung cancer cells are from a A549 cell line.

18. The method as claimed in claim 17, wherein the half maximal inhibitory concentration (IC₅₀) for the lung cancer cell line A549 is 2.34 ng/ml.

19. The method as claimed in claim 12, wherein the prostate cancer cells are from a LNCaP cell line or a DU-145 cell line.

20. The method as claimed in claim 19, wherein the half maximal inhibitory concentration (IC₅₀) for the prostate cancer cell line LNCaP is 0.31 ng/ml.

21. The method as claimed in claim 19, wherein the half maximal inhibitory concentration (IC₅₀) for the prostate cancer cell line DU-145 is 0.28 ng/ml.

22. A pharmaceutical composition used for inhibiting the growth of cancer cells, which comprises an active dose of the compound as claimed in claim 12 and a pharmaceutically-acceptable carrier, wherein said cancer cells are selected from the group consisting of hepatic cancer, lung cancer or prostate cancer.
